# NOUVEAU FASCICULE DE BREVET EUROPEEN

(11) **EP 2 651 372 B2**
(45) Date de publication et mention de la décision concernant l'opposition: **25.10.2023**
(45) Mention de la délivrance du brevet: 14.10.2020
(21) Numéro de dépôt: 11817336.8
(22) Date de dépôt: 14.12.2011
(51) Int. Cl.: A61K 8/04, A61K 8/73, A61Q 5/06, A61K 8/00, A61K 8/34, A61K 8/81, A45D 34/00, A61K 8/85

(54) **DISPOSITIF AEROSOL A DEUX COMPARTIMENTS COMPRENANT UNE COMPOSITION DE COIFFAGE, ALCOOLIQUE OU HYDROALCOOLIQUE, ET PROCEDE DE COIFFAGE**
AEROSOLVORRICHTUNG MIT ZWEI BEHÄLTERN WELCHE EINE (HYDRO)ALKOHOLISCHE HAARSTYLINGZUSAMMENSTZUNG ENTHÄLT SOWIE METHODE ZUM HAARSTYLING
AEROSOL DEVICE HAVING TWO COMPARTMENTS INCLUDING AN ALCOHOLIC OR HYDROALCOHOLIC HAIRSTYLING COMPOSITION, AND HAIRSTYLING METHOD

(30) Priorité: 14.12.2010 FR 1060475; 14.12.2010 FR 1060476; 14.12.2010 FR 1060477; 14.01.2011 US 201161432796 P; 19.01.2011 US 201161434064 P
(43) Date de publication de la demande: 23.10.2013
(73) Titulaire: L'OREAL, 75008 Paris (FR)
(72) Inventeur: LE BOURHIS, François, F-93300 Aubervilliers (FR); GAWTREY, Jonathan, F-92100 Boulogne (FR)
(74) Mandataire: Casalonga
(86) Numéro de dépôt international: PCT/FR2011/052988
(87) Numéro de publication internationale: WO 2012/080661

(56) Documents cités:
- EP-A1- 0 791 351
- EP-A1- 1 366 747
- EP-A1- 1 366 747
- EP-A1- 1 366 747
- EP-A1- 1 366 751
- EP-A1- 1 366 751
- EP-A1- 1 366 751
- EP-A2- 1 291 004
- EP-A2- 1 291 004
- WO-A1-97/17052
- WO-A2-2010/133660
- WO-A2-2010/133660
- WO-A2-2010/133660
- DE-A1- 2 619 354
- DE-A1- 2 849 592
- DE-A1- 10 237 737
- FR-A1- 2 858 979
- FR-A1- 2 873 577
- FR-A1- 2 873 577
- FR-A1- 2 873 577
- US-A- 3 715 428
- US-A- 3 715 428
- US-A- 3 715 428
- US-A- 5 804 166
- US-A- 5 804 166
- WOOD C: "POLYMERE FUER FRISUREN", CHEMIE IN UNSERER ZEIT., VERLAG CHEMIE, WEINHEIM., DE, vol. 36, no. 01, 1 January 2002 (2002-01-01), pages 44-52, XP001093907, DE ISSN: 0009-2851, DOI: 10.1002/1521-3781(200202)36:1<44::AID-C IUZ44>3.0.CO;2-Z

## Description

La présente invention est relative à un dispositif aérosol à deux compartiments comprenant une composition de coiffage et un gaz comprimé particulier, et à un procédé de coiffage.

Les compositions de coiffage, telles que les laques et les sprays, conditionnées sous forme de spray aérosol sont généralement composées d'une phase liquide comprenant, dans un milieu cosmétiquement acceptable aqueux, alcoolique ou hydroalcoolique, au moins un polymère de fixation, et d'un agent propulseur qui est un gaz liquéfié sous pression réduite ou dissous dans la phase liquide.

D'autres dispositifs aérosols comprenant une telle phase liquide existent, mais ils comprennent des gaz comprimés pour assurer la propulsion de la phase liquide. Or ces dispositifs à gaz comprimés présentent l'inconvénient d'une perte de pression des gaz au cours du temps. En effet, on observe une fuite des gaz comprimés au cours du temps ou lors d'une mauvaise utilisation du récipient, c'est-à-dire lorsqu'il se retrouve la tête en bas.

Cet inconvénient entraîne une propulsion de moins en moins bonne de la phase liquide au cours du temps et par conséquent une répartition de moins en moins bonne de la phase liquide sur les cheveux.

Un autre facteur pouvant conduire à une mauvaise répartition de la phase liquide à partir de ces dispositifs est la qualité du spray qui se dégrade lorsque l'on cherche à obtenir une fixation la plus importante possible, c'est-à-dire lorsque l'on cherche à augmenter la quantité de polymère que l'on peut dissoudre dans ladite phase liquide. D'autres diapositifs aérosol, notamment à deux compartiments avec un gaz comprimé, ont aussi été décrits dans l'art antérieur pour l'application de compostions coiffantes pour cheveux (voir FR 2 858 979 A1).

La demanderesse a fait la découverte surprenante que la séparation par voie mécanique de la phase liquide du gaz comprimé assurant la propulsion, et l'utilisation d'un gaz comprimé particulier et d'un composé choisi parmi un polymère anionique carboxylique sans motif uréthane, dans la composition propulsée permettait de résoudre les problèmes de fuite et de répartition de la phase liquide sur les cheveux. Cette combinaison particulière permet ainsi d'obtenir une fixation et/ou une mise en forme avec un niveau de fixation qui ne peut être atteint habituellement avec ce type de produit.

L'invention a donc pour objet un dispositif aérosol à deux compartiments tel que décrit ci-dessous.

Un autre objet de la présente invention consiste en un procédé de coiffage mettant en œuvre ce dispositif.

D'autres objets, caractéristiques, aspects et avantages de l'invention apparaîtront encore plus clairement à la lecture de la description et des divers exemples qui suivent.

Le dispositif aérosol à deux compartiments selon l'invention comprend :
(a) dans un premier compartiment, une composition de coiffage comprenant au moins composé (A) choisi parmi :
   un polymère anionique carboxylique sans motif uréthane dans un milieu alcoolique ou hydroalcoolique qui comprend de 20 à 98 % en poids, par rapport au poids total de ladite composition, d'un alcool monohydrique en C₁-C₄ et
(b) dans un deuxième compartiment, un gaz comprimé choisi parmi l'air, l'azote, le gaz carbonique et leurs mélanges, l'air étant particulièrement préféré,
   à condition que lorsque la composition de coiffage comprend en outre un ou plusieurs polymères fixants additionnels, ceux-ci ne soient pas des polyuréthanes.

Ledit gaz comprimé est utilisé de préférence sous une pression comprise entre 1 et 12 bars, mieux encore comprise entre 9 et 11 bars.

Dans ce qui suit ou précède l'expression « au moins un(e) » est équivalente à « un(e) ou plusieurs ».

### POLYMERE FIXANT ANIONIQUE CARBOXYLIQUE NON POLYURETHANE (sans motif uréthane)

Les polymères fixants anioniques pouvant être utilisés selon l'invention sont des polymères comportant des groupements dérivés d'acide carboxylique, et qui ont de préférence une masse moléculaire moyenne en nombre comprise entre environ 500 et 5 000 000.

Les groupements carboxyliques sont apportés par des monomères mono- ou diacides carboxyliques insaturés tels que ceux répondant à la formule : 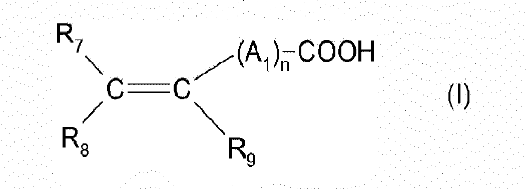 dans laquelle n est un nombre entier de 0 à 10, Al désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R7 désigne un atome d'hydrogène, un groupement phényle ou benzyle, R8 désigne un atome d'hydrogène, un groupement alkyle inférieur ou carboxyle, R9 désigne un atome d'hydrogène, un groupement alkyle inférieur, un groupement -CH2-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques peuvent être :
A) les copolymères d'acide acrylique et d'acrylamide tels que ceux vendus sous la forme de leurs sels de sodium sous les dénominations RETEN 421, 423 ou 425 par la Société HERCULES, les sels de sodium des acides polyhydroxycarboxyliques ;
B) les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans les demandes FR 1 222 944 et DE 2 330 956, les copolymères de ce type comportant dans leur chaîne un motif acrylamide éventuellement N-alkylé et/ou hydroxyalkylé tels que décrits notamment dans les demandes de brevets LU 75370 et LU 75371 ou proposés sous la dénomination QUADRAMER par la Société AMERICAN CYANAMID. On peut également citer les terpolymères acide méthacrylique/acrylate d'éthyle/acrylate de tertiobutyle tels que le produit commercialisé sous la dénomination LUVIMER^{®} 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD^{®} DR 25 par la société AMERCHOL.
   On peut aussi citer les polymères du type FIXATE G 100.
C) les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les demandes FR 1 222 944, FR 1 580 545, FR 2 265 782, FR 2 265 781, FR 1 564 110 et FR 2 439 798. Des produits commerciaux entrant dans cette classe sont les résines 28-29-30, 26-13-14 et 28-13-10 commercialisées par la société National Starch.
D) les copolymères d'acides ou d'anhydrides carboxyliques monoinsaturés en C4-C8 choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les demandes US 2,047,398, US 2,723,248, US 2,102,113, et GB 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
      les fonctions anhydrides de ces copolymères étant éventuellement monoestérifiées ou monoamidifiées.
   Ces polymères sont par exemple décrits dans les demandes FR 2 350 384 et FR 2 357 241 de la demanderesse.
E) les polyacrylamides comportant des groupements carboxylates.

De préférence, les polymères fixants anioniques carboxyliques sont choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX^{®} A par la société BASF.

Les polymères fixants de l'invention peuvent également être des polymères à squelettes siliconés porteurs de greffons non siliconés ou des polymères non siliconés à greffons siliconés, porteurs de groupements carboxyliques, tels que le polymère VS80 de la société 3M.

Parmi les polymères fixants anioniques cités ci-dessus, on préfère plus particulièrement les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF.

Lorsqu'ils sont présents, le ou les polymères fixants anioniques carboxyliques sont de préférence présents en une quantité allant de 0,5 à 20 % en poids, mieux encore de 1 à 12 % en poids par rapport au poids total de la composition de coiffage.

### POLYESTER SULFONIQUE LINEAIRE

La composition selon l'invention peut comprendre en outre au moins un polyester sulfonique linéaire.

Par polyester sulfonique on entend un polyester comprenant un ou plusieurs groupements sulfonique, sous forme libre ou partiellement voire totalement salifiés

Ce polyester linéaire sulfonique est soluble dans l'eau ou hydrodispersible

Par polyester sulfonique linéaire hydrodispersible, on entend tout polyester sulfonique présentant une aptitude à former une dispersion, c'est-à-dire un système biphasique où la première phase est formée de particules finement divisées et distribuées uniformément dans la seconde phase qui est la phase continue.

Par polyester sulfonique, on entend de préférence des copoylesters obtenus par polycondensation d'au moins un acide dicarboxylique ou d'un de ses esters, d'au moins un diol et d'au moins un composé difonctionnel sulfoaryldicarboxylique substitué sur le noyau aromatique par un groupe -SO₃M dans lequel M représente un atome d'hydrogène ou un ion métallique tel que Na⁺, Li⁺ ou K⁺.

Les polyesters sulfoniques linéaires de l'invention présentent généralement une masse moléculaire moyenne en poids comprise entre environ 1 000 et 60 000, et de préférence de 4 000 à 20 000, telle que déterminée par chromatographie par perméation de gel (ou GPC).

La température de transition vitreuse (Tg) de ces polyesters sulfoniques est généralement comprise dans l'intervalle allant de 10 °C à 100 °C. De préférence, la Tg du ou des polyesters utilisés est supérieure ou égale à 50°C.

La température de transition vitreuse (Tg) est mesurée par analyse enthalpique différentielle (DSC, *differential scanning calorimetry*) selon la norme ASTM D3418-97.

De préférence les polyesters linéaires de l'invention sont hydrodispersibles.

Les polyesters sulfoniques linéaires de l'invention sont décrits plus en détail dans les demandes de brevet US 3 734 874, US 3 779 993, US 4 119 680, US 4 300 580, US 4 973 656, US 5 660 816, US 5 662 893, et US 5 674 479.

Les polyesters sulfoniques utilisés de préférence dans l'invention comprennent au moins des motifs dérivés d'acide isophtalique, de sel d'acide sulfoaryle-dicarboxylique et de diéthylèneglycol, et plus particulièrement les polyesters sulfoniques utilisés dans l'invention sont obtenus à partir d'acide isophtalique, de sel de sodium de l'acide sulfoisophtalique, de diéthylèneglycol et de 1,4-cyclohexaneméthanol.

A titre d'exemples de polyester sulfonique, on peut notamment citer ceux connus sous le nom INCI Diglycol/CHDM/Isophtalates/SIP, et vendus sous les dénominations commerciales"Eastman AQ polymer" (AQ35S, AQ38S, AQ55S, AQ48 Ultra) par la société Eastman Chemical.

De préférence, la Tg du ou des polyesters utilisés est supérieure ou égale à 50°C.

Lorsqu'ils sont présents, le ou les polyesters sulfoniques linéaires sont de préférence présents en une quantité allant de 0,5 à 20 % en poids, mieux encore de 1 à 12 % en poids par rapport au poids total de la composition de coiffage.

### COMPOSE CELLULOSIQUE

La composition selon l'invention peut comprendre en outre au moins un composé cellulosique.

Par composé cellulosique, on entend selon l'invention tout composé polysaccharidique possédant dans sa structure des enchaînements de résidus glucose unis par des liaisons β-1,4.

Les composés cellulosiques peuvent être les celluloses proprement dites y compris sous une forme microcristalline et les éthers de cellulose. Les éthers de cellulose sont choisis de préférence parmi les éthers non ioniques, anioniques ou cationiques.

Parmi les éthers de cellulose non ioniques, on peut citer les alkylcelluloses telles que les méthylcelluloses et les éthylcelluloses ou les hydroxyalkylcelluloses telles que les hydroxyméthylcelluloses, les hydroxyéthylcelluloses et les hydroxypropylcelluloses et les celluloses mixtes hydroxyalkyl-alkylcelluloses telles que les hydroxypropyl-méthylcelluloses, les hydroxyéthyl-méthylcelluloses, les hydroxyéthyl-éthylcelluloses et les hydroxybutyl-méthylcelluloses.

Parmi les composés anioniques, on peut citer les carboxyalkylcelluloses et leurs sels. A titre d'exemple, on peut citer les carboxyméthylcelluloses, les carboxyméthylméthylcelluloses et les carboxyméthylhydroxyéthylcelluloses et leurs sels de sodium.

Parmi les composés cationiques, on peut citer les hydroxyéthylcelluloses quaternisées réticulées ou non. Le quaternisant peut être notamment le chlorure de glycidyltriméthylammonium ou une amine grasse telle que la laurylamine ou la stéarylamine. Comme autre cellulose cationique, on peut citer l'hydroxyéthylcellulosehydroxypropyltriméthylammonium.

De préférence le composé cellulosique de l'invention est non-ionique.

Encore plus préférentiellement le composé cellulosique est une hydroxypropylcellulose.

Lorsqu'ils sont présents, le ou les composés cellulosiques sont de préférence présents en une quantité allant de 0,5 à 20 % en poids, mieux encore de 1 à 12 % en poids par rapport au poids total de la composition de coiffage.

### MILIEU COSMETIQUE

Les compositions de l'invention comprennent au moins un alcool monohydrique en C₁₋₄, à raison d'une teneur de 20 à 98 % en poids par rapport au poids de ladite composition.

Par monohydrique, on entend un alcool ne comprenant qu'une fonction hydroxy.

Les alcools monohydriques de l'invention sont de préférence choisis parmi l'éthanol, l'isopropanol, le n propanol, le tertiobutanol ou leurs mélanges.

De préférence ils sont choisis parmi l'éthanol, l'isopropanol ou leurs mélanges.

Encore plus préférentiellent, l'alcool monohydrique en C₁₋₄ est l'éthanol.

Le milieu comprend de 20 à 98 % en poids par rapport au poids total de ladite composition, d'un ou plusieurs alcools monohydriques en C₁₋₄.

Dans un milieu hydroalcoolique, la proportion d'eau peut varier de 1 à 95 %, et de préférence de 10 à 90 % du poids total de la composition.

Selon un mode de réalisation particulier, la composition selon l'invention peut comprendre en outre un ou plusieurs polymères fixants additionnels.

Par « polymère fixant », on entend au sens de la présente invention tout polymère permettant de conférer une forme ou de maintenir une forme ou une coiffure donnée.

Les polymères fixants synthétiques additionnels sont généralement choisis parmi les polymères cationiques, amphotères, non ioniques et les polymères anioniques et leurs mélanges différents du ou des composés (A) déjà contenus dans la composition.

Par « polymère cationique », on entend au sens de la présente invention, tout polymère comprenant des groupements cationiques et/ou des groupements ionisables en groupements cationiques.

Les polymères fixants cationiques peuvent être choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 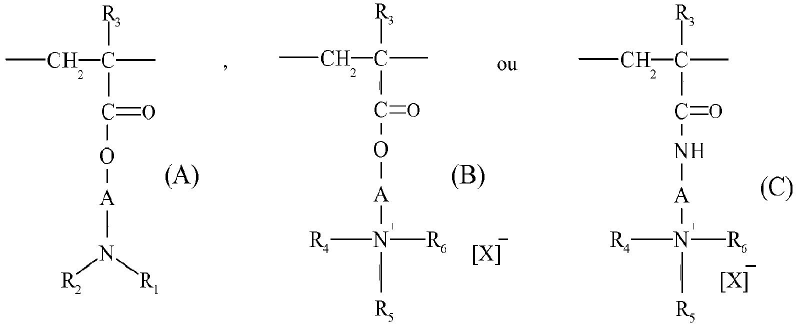 dans lesquelles : R1 et R2, identiques ou différents, représentent chacun un atome d'hydrogène ou un groupe alkyle ayant de 1 à 6 atomes de carbone ; R3 désigne un atome d'hydrogène ou un groupe CH3 ; A est un groupe alkyle linéaire ou ramifié, comportant de 1 à 6 atomes de carbone ou un groupe hydroxyalkyle comportant de 1 à 4 atomes de carbone ;
   R4, R5, R6, identiques ou différents, représentent un groupe alkyle ayant de 1 à 18 atomes de carbone ou un groupe benzyle ; X désigne un anion méthosulfate ou un halogénure tel que chlorure ou bromure.
   Ces copolymères contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C1-4), des groupes dérivés des acides acryliques ou méthacryliques ou de leurs esters, de vinyllactames tels que la vinylpyrrolidone ou le vinylcaprolactame, d'esters vinyliques. Ainsi, parmi ces copolymères, on peut citer :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC^{®} par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
   - les copolymères d'acrylamide et de méthosulfate de méthacryloyloxyéthyltriméthylammonium, tels que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination « GAFQUAT^{®} » par la société ISP comme, par exemple, « GAFQUAT^{®} 734 » ou « GAFQUAT^{®} 755 », ou bien les produits dénommés « COPOLYMER^{®} 845, 958 et 937 ». Ces polymères sont décrits en détail dans les demandes FR 2 077 143 et FR 2 393 573,
   - les terpolymères méthacrylate de diméthylaminoéthyle/vinylcaprolactame/vinylpyrrolidone tels que le produit commercialisé sous la dénomination GAFFIX^{®} VC 713 par la société ISP, et
   - les copolymères vinylpyrrolidone/méthacrylamide de diméthylaminopropyle quaternisé tels que notamment le produit commercialisé sous la dénomination « GAFQUAT^{®} HS 100 » par la société ISP ;
(2) les copolymères quaternaires de vinylpyrrolidone et de vinylimidazole.

Les polymères fixants amphotères peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes ;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus peuvent être choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet US 3,836,537,
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement utilisés sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   Il s'agit plus particulièrement des copolymères dont la dénomination CTFA (4ème Ed., 1991) est octylacrylamide/acrylates/ butylaminoethyl methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de polyaminoamides de formule générale : ⁅co-R₁₀-co-co-z⁆ (XVII) dans laquelle R10 représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 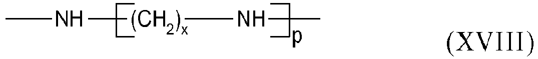
      où x=2 et p=2 ou 3, ou bien x=3 et p=2
      ce groupe dérivant de la diéthylène-triamine, de la triéthylène-tétraamine ou de la dipropylène-triamine ;
   b) dans les proportions de 0 à 40 % en moles, le groupe (XVIII) ci-dessus, dans lequel x=2 et p=1 et qui dérive de l'éthylène-diamine, ou le groupe dérivant de la pipérazine : 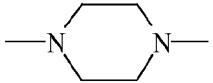
   c) dans les proportions de 0 à 20 % en moles, le groupe -NH-(CH2)6-NH- dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 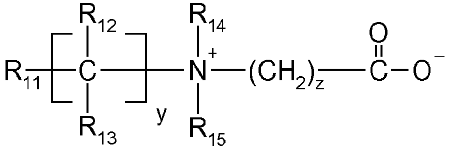 dans laquelle R11 désigne un groupement insaturé polymérisable tel qu'un groupement acrylate, méthacrylate, acrylamide ou méthacrylamide, y et z représentent un nombre entier de 1 à 3, R12 et R13 représentent un atome d'hydrogène, un groupe méthyle, éthyle ou propyle, R14 et R15 représentent un atome d'hydrogène ou un groupe alkyle de telle façon que la somme des atomes de carbone dans R14 et R15 ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonioéthylméthacrylate de méthyle, tels que le produit vendu sous la dénomination DIAFORMER Z301 par la société SANDOZ.
(5) les polymères dérivés du chitosane comportant des motifs monomères répondant aux formules suivantes : 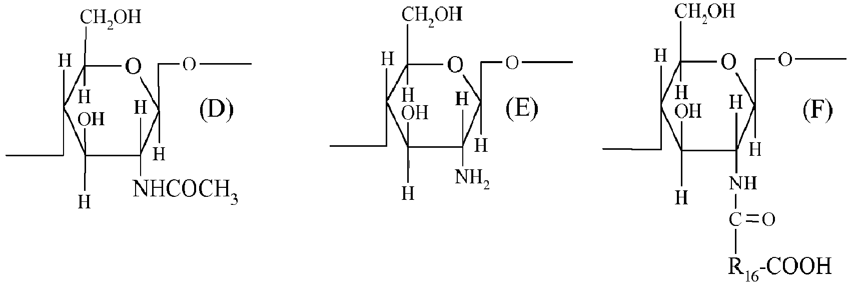 le motif (D) étant présent dans des proportions comprises entre 0 et 30 %, le motif (E) dans des proportions comprises entre 5 et 50 % et le motif (F) dans des proportions comprises entre 30 et 90 %, étant entendu que dans ce motif (F), R16 représente un groupe de formule : 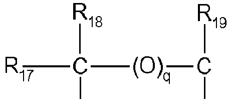 dans laquelle si q=0, R17, R18 et R19, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R17, R18 et R19 étant dans ce cas un atome d'hydrogène ;
   ou si q=1, R17, R18 et R19 représentent chacun un atome d'hydrogène, ainsi que les sels formés par ces composés avec des bases ou des acides.
(6) les polymères comportant des motifs répondant à la formule générale (XIX) qui sont, par exemple, décrits dans le brevet FR 1 400 366 : 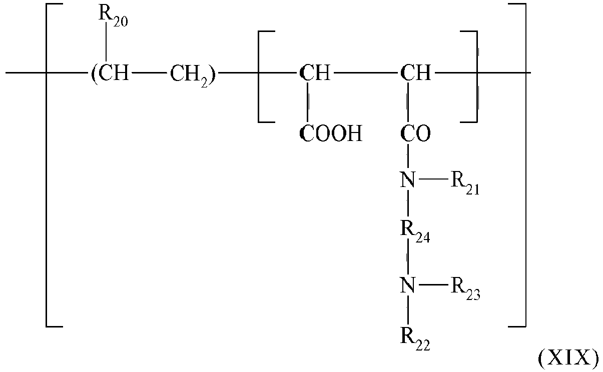 dans laquelle R20 représente un atome d'hydrogène, un groupe CH30, CH3CH2O, phényle, R21 désigne un atome d'hydrogène ou un groupe alkyle inférieur tel que méthyle, éthyle, R22 désigne un atome d'hydrogène ou un groupe alkyle inférieur en C1-C6 tel que méthyle, éthyle, R23 désigne un groupe alkyle inférieur en C1-C6 tel que méthyle, éthyle ou un groupe répondant à la formule : -R24-N(R22)2, R24 représentant un groupement -CH2-CH2-, -CH2-CH2-CH2-, -CH2-CH(CH3)-, R22 ayant les significations mentionnées ci-dessus.
(7) les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (XX)

      où D désigne un groupe 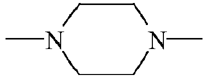 et X désigne le symbole E ou E', E ou E' identiques ou différents désignant un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      D-X-D-X- (XXI')

      où D désigne un groupe 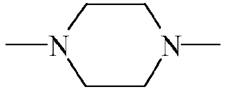 et X désigne le symbole E ou E' et au moins une fois E' ; E ayant la signification indiquée ci-dessus et E' étant un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères décrits ci-dessus, les plus particulièrement préférés sont ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthylcarboxyméthylammonioéthylméthacrylate de méthyle vendu par exemple sous la dénomination DIAFORMER^{®} Z301 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILlTH^{®} LDM 6911, MOWILlTH^{®} DM 611 et MOWILlTH^{®} LDM 6070 proposés par la société HOECHST, les produits RHODOPAS^{®} SD 215 et RHODOPAS^{®} DS 910 proposés par la société RHODIA CHIMIE ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame tels que la polyvinylpyrrolidone ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyle des polymères non ioniques mentionnés ci-dessus ont, de préférence, de 1 à 6 atomes de carbone.

Les polymères fixants additionnels anioniques peuvent être des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 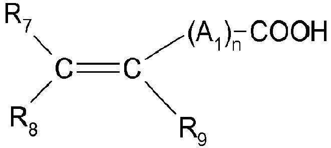 dans laquelle n est un nombre entier de 0 à 10, A1 désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R7 désigne un atome d'hydrogène, un groupement phényle ou benzyle, R8 désigne un atome d'hydrogène, un groupement alkyle inférieur ou carboxyle, R9 désigne un atome d'hydrogène, un groupement alkyle inférieur, un groupement -CH2-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques peuvent être :
A) les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans les demandes FR 1 222 944 et DE 2 330 956, les copolymères de ce type comportant dans leur chaîne un motif acrylamide éventuellement N-alkylé et/ou hydroxyalkylé tels que décrits notamment dans les demandes de brevets LU 75370 et LU 75371 ou proposés sous la dénomination QUADRAMER par la Société AMERICAN CYANAMID. On peut également citer les terpolymères acide méthacrylique/acrylate d'éthyle/acrylate de tertiobutyle tels que le produit commercialisé sous la dénomination LUVIMER^{®} 100 P par la société BASF.
   On peut aussi citer les copolymères acide méthacrylique/acide acrylique/acrylate d'éthyle/méthacrylate de méthyle en dispersion aqueuse, commercialisé sous la dénomination AMERHOLD^{®} DR 25 par la société AMERCHOL.
   On peut aussi citer les polymères du type FIXATE G 100.
C) les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les demandes FR 1 222 944, FR 1 580 545, FR 2 265 782, FR 2 265 781, FR 1 564 110 et FR 2 439 798. Des produits commerciaux entrant dans cette classe sont les résines 28-29-30, 26-13-14 et 28-13-10 commercialisées par la société National Starch.
D) les copolymères d'acides ou d'anhydrides carboxyliques monoinsaturés en C4-C8 choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les demandes US 2,047,398, US 2,723,248, US 2,102,113, et GB 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,

   les fonctions anhydrides de ces copolymères étant éventuellement monoestérifiées ou monoamidifiées.
   Ces polymères sont par exemple décrits dans les demandes FR 2 350 384 et
   FR 2 357 241 de la demanderesse.
E) les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique. Ils sont différents des polyesters sulfoniques linéaires de l'invention.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous les dénominations Flexan^{®} 500 et Flexan^{®} 130 par National Starch. Ces composés sont décrits dans le brevet FR 2 198 719.

- les sels d'acides polyacrylamide-sulfoniques tels que ceux mentionnés dans le brevet US 4,128,631, et plus particulièrement l'acide polyacrylamidoéthylpropane-sulfonique vendu sous la dénomination COSMEDIA POLYMER HSP 1180 par Henkel.
- les polyesters sulfoniques ramifiés tels que le produit proposé sous la dénomination AQ 1350.

De préférence, les polymères fixants anioniques sont choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX^{®} A par la société BASF.

Les polymères fixants de l'invention peuvent également être des polymères à squelettes siliconés porteurs de greffons non siliconés ou des polymères non siliconés à greffons siliconés, tels que le polymère VS80 de la société 3M.

Parmi les polymères fixants anioniques cités ci-dessus, on préfère plus particulièrement les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF.

Selon l'invention, le ou les polymères fixants additionnels ne sont pas des polyuréthanes.

De préférence le ou les polymères fixants additionnels sont anioniques ou non ioniques.

Lorsqu'ils sont présents le ou les polymères fixants additionnels représentent de 0,1 à 20% en poids, mieux de 0,5 à 10% en poids par rapport au poids total de la composition.

La composition de coiffage peut comprendre en outre des additifs tels que des silicones sous forme soluble, dispersée, micro-dispersée, des actifs traitants, des agents hydratants comme le glycérol, des filtres UV, des acides, des bases, des agents plastifiants, des agents solubilisants, des agents conservateurs, des vitamines et des provitamines, des colorants, des pigments, des agents tensioactifs anioniques, cationiques, non ioniques ou amphotères, des parfums, des agents anti-corrosion, et leurs mélanges.

L'homme de métier veillera à choisir les éventuels additifs et leur quantité de manière à ce qu'ils ne nuisent pas aux propriétés des compositions de la présente invention.

Ces additifs sont notamment présents dans la composition selon l'invention en une quantité allant de 0 à 20 % en poids par rapport au poids total de la composition.

De préférence, le dispositif aérosol à deux compartiments est constitué par un bidon aérosol externe comportant une poche interne soudée hermétiquement à une valve. La composition est introduite dans la poche interne et un gaz comprimé est introduit entre la poche et le bidon à une pression suffisante pour faire sortir le produit sous forme d'un spray à travers l'orifice d'une buse. Un tel dispositif est commercialisé sous le nom EP SPRAY par la société EP-SPRAY SYSTEM SA.

Les dispositifs aérosols de l'invention sont de préférence des laques pour cheveux.

La présente invention concerne également un procédé de coiffage qui consiste en ce que l'on vaporise la composition de coiffage contenue dans le dispositif aérosol selon l'invention, sur les cheveux mouillés ou non et que généralement on sèche les cheveux avec un dispositif chauffant (sèche cheveux, casque,...) ou qu'on les laisse sécher spontanément.

Les exemples suivants sont donnés à titre illustratif de la présente invention sans caractère limitatif.

Toutes les quantités sont indiquées en pour cent en poids en matières actives par rapport au poids total de la composition.

On a préparé les compositions de coiffage à partir des ingrédients suivants :

### Exemple 1 (selon l'invention)

| | |
|---|---|
| Copolymère acétate de vinyle / acide crotonique (90/10) (Luviset CA 66 fournisseur BASF) | 5.00 |
| Amino méthylpropanol | Qsp 95-100 % de neutralisation |
| Parfum | 0.10 |
| Alcool éthylique qsp | 100 |

### Exemple 2 (référence)

| | |
|---|---|
| POLYESTER-5 (AQ 48 ULTRA POLYMER fournisseur EASTMAN ) (Exprimé en poids de polymère) | 5.00 |
| Eau déminéralisée | 40 |
| Parfum | 0.10 |
| Alcool éthylique qsp | 100 |

### Exemple 3 (référence)

| | Composition A | Composition B |
|---|---|---|
| Hydroxypropyl cellulose (KLUCEL EF PHARM de ASHLAND) | 3 | 3 |
| Eau déminéralisée | 18 | 18 |
| Glycérol | - | 0,3 |
| Alcool éthylique qsp | qsp100 | qsp100 |

On a introduit chacune des compositions préparées ci-dessus dans un dispositif de distribution aérosol commercialisé sous le nom EP SPRAY par la société EP SPRAY SYSTEM S.A. décrit ci-dessus. Une valve de référence 6001 format D6 est fixée sur un bidon aérosol classique, et le diffuseur est un diffuseur à buse tourbillonnaire.

La poche est remplie avec la composition comme indiqué ci-dessus. De l'air comprimé est introduit entre la poche et le bidon.

On a vaporisé les compositions sur des cheveux secs. La pulvérisation se fait sous forme d'un spray doux de très bonne qualité.

On obtient, après séchage, une chevelure présentant un très bon maintien.

Le dispositif aérosol ne se bouche pas au fur et à mesure des applications.

## Revendications

1. Dispositif aérosol à deux compartiments, comprenant :
a) dans un premier compartiment, une composition de coiffage comprenant au moins un composé (A) choisi parmi un polymère anionique carboxylique sans motif uréthane dans un milieu alcoolique ou hydroalcoolique qui comprend de 20 à 98 % en poids par rapport au poids de ladite composition, d'un alcool monohydrique en C₁-C₄, et
b) dans un deuxième compartiment, un gaz comprimé choisi parmi l'air, l'azote, le gaz carbonique et leurs mélanges,
à condition que lorsque la composition de coiffage comprend en outre un ou plusieurs polymères fixants additionnels, ceux-ci ne soient pas des polyuréthanes.

2. Dispositif aérosol selon la revendication 1, **caractérisé en ce que** le gaz comprimé est l'air.

3. Dispositif aérosol selon la revendication 1 ou 2, **caractérisé en ce que** la pression du gaz comprimé est comprise entre 1 et 12 bars.

4. Dispositif aérosol selon la revendication 3, **caractérisé en ce que** la pression du gaz comprimé est comprise entre 9 et 11 bars.

5. Dispositif selon l'une quelconque des revendications précédentes, **caractérisé en ce que** le polymère anionique est choisi parmi :
A) les copolymères d'acide acrylique et d'acrylamide, les sels de sodium des acides polyhydroxycarboxyliques ;
B) les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés.
C) les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique.
D) les copolymères d'acides ou d'anhydrides carboxyliques monoinsaturés en C4-C8 choisis parmi :
- les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées.
- les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
E) les polyacrylamides comportant des groupements carboxylates.

6. Dispositif aérosol selon l'une quelconque des revendications 1 à 5, caractérisé en ce le polymère anionique est choisi parmi les copolymères d'acide acrylique tels que les terpolymères acide acrylique/acrylate d'éthyle/N-tertiobutylacrylamide, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié, les copolymères d'acide méthacrylique et de méthacrylate de méthyle, les copolymères d'acide méthacrylique et d'acrylate d'éthyle et les copolymères acétate de vinyle/acide crotonique et les copolymères acétate de vinyle/acide crotonique greffés par du polyéthylèneglycol.

7. Dispositif aérosol selon l'une quelconque des revendications précédentes, **caractérisé en ce que** le ou les composés (A) choisis parmi un polymère anionique carboxylique sans motif uréthane sont présents en une quantité allant de 0,5 à 20 % en poids, mieux encore de 1 à 12 % en poids par rapport au poids total de la composition.

8. Dispositif aérosol selon l'une quelconque des revendications précédentes, **caractérisé en ce que** l'alcool monohydrique est de préférence choisis parmi l'éthanol, l'isopropanol, le n propanol, le tertiobutanol ou leurs mélanges de préférence parmi l'éthanol, l'isopropanol ou leurs mélanges encore plus préférentiellement est l'éthanol.

9. Dispositif aérosol selon l'une quelconque des revendications précédentes, **caractérisé en ce que** la composition comprend de 1 à 95 %, et de préférence de 10 à 90 % d'eau en poids par rapport au poids total de la composition.

10. Utilisation du dispositif aérosol selon l'une quelconque des revendications précédentes pour une laque pour cheveux.

11. Procédé de coiffage, **caractérisé en ce que** l'on vaporise la composition de coiffage contenue dans le dispositif aérosol selon l'une quelconque des revendications 1 à 9, sur les cheveux mouillés ou non.

## Patentansprüche

1. Aerosolvorrichtung mit 2 Kompartimenten, umfassend:
a) in einem ersten Kompartiment eine Frisierzusammensetzung, umfassend mindestens eine Verbindung (A), ausgewählt aus einem anionischen Carboxylpolymer ohne Urethanmotiv, in einem alkoholischen oder wässrig-alkoholischen Medium, das 20 bis 98 Gew.-%, bezogen auf das Gewicht der Zusammensetzung, eines einwertigen C₁-C₄-Alkohols umfasst, und
b) in einem zweiten Kompartiment ein komprimiertes Gas, ausgewählt aus Luft, Stickstoff, Kohlendioxid und ihren Gemischen,
vorausgesetzt, dass, wenn die Frisierzusammensetzung außerdem ein oder mehrere zusätzliche fixierende Polymere umfasst, es sich hierbei nicht um Polyurethane handelt.

2. Aerosolvorrichtung nach Anspruch 1, **dadurch gekennzeichnet, dass** es sich bei dem komprimierten Gas um Luft handelt.

3. Aerosolvorrichtung nach Anspruch 1 oder 2, **dadurch gekennzeichnet, dass** der Druck des komprimierten Gases zwischen 1 und 12 bar beträgt.

4. Aerosolvorrichtung nach Anspruch 3, **dadurch gekennzeichnet, dass** der Druck des komprimierten Gases zwischen 9 und 11 bar beträgt.

5. Vorrichtung nach einem der vorhergehenden Ansprüche, **dadurch gekennzeichnet, dass** das anionische Polymer ausgewählt ist aus:
A) Copolymeren von Acrylsäure und Acrylamid, Natriumsalzen von Polyhydroxycarbonsäuren;
B) Copolymeren von Acryl- oder Methacrylsäure mit einem monoethylenischen Monomer, wie Ethylen, Styrol, Vinylester, Acrylsäure- oder Methacrylsäureester, gegebenenfalls gepfropft auf ein Polyalkylenglykol, wie Polyethylenglykol, und gegebenenfalls vernetzt;
C) Copolymeren von Crotonsäure, wie diejenigen, die in ihrer Kette Vinylacetat- oder Vinylpropionatmotive enthalten, und gegebenenfalls anderen Monomeren, wie Allyl- oder Methallylester, Vinylether oder Vinylester einer gesättigten, geraden oder verzweigten Carbonsäure mit langer Kohlenwasserstoffkette, wie diejenigen, die mindestens 5 Kohlenstoffatome enthalten, wobei diese Polymere gegebenenfalls gepfropft oder vernetzt sein können, oder auch einem anderen Vinyl-, Allyl- oder Methallylester-Monomer einer α- oder β-zyklischen Carbonsäure;
D) Copolymeren einfach ungesättigter C4-C8-Carbonsäuren oder -Carbonsäureanhydriden, ausgewählt aus:
- Copolymeren, umfassend (i) ein(e) oder mehrere Malein-, Fumar-, Itaconsäuren oder -säureanhydride und (ii) mindestens ein Monomer, ausgewählt aus Vinylestern, Vinylethern, Vinylhalogeniden, Phenylvinylderivaten, Acrylsäure und ihren Estern, wobei die Anhydridfunktionen dieser Copolymere gegebenenfalls monoverestert oder monoamidiert sind;
- Copolymeren, umfassend (i) ein oder mehrere Maleinsäure-, Citraconsäure-, Itaconsäureanhydridmotive und (ii) ein oder mehrere Monomere, ausgewählt aus Allyl- oder Methallylestern, die gegebenenfalls ein oder mehrere Acrylamid-, Methacrylamid-, α-Olefin-, Acryl- oder Methacrylester-, Acryl- oder Methacrylsäure- oder Vinylpyrrolidongruppen in ihrer Kette enthalten,
wobei die Anhydridfunktionen dieser Copolymere gegebenenfalls monoverestert oder monoamidiert sind;
E) Polyacrylamiden, die Carboxylatgruppen tragen.

6. Aerosolvorrichtung nach einem der Ansprüche 1 bis 5, **dadurch gekennzeichnet, dass** das anionische Polymer ausgewählt ist aus Acrylsäure-Copolymeren, wie Acrylsäure/Ethylacrylat/N-tert-Butylacrylamid-Terpolymere, von Crotonsäure abgeleiteten Copolymeren, wie Vinylacetat/Vinyl-tert-butylbenzoat/Crotonsäure-Terpolymere und Crotonsäure/Vinylacetat/Vinylneododecanoat-Terpolymere, von Malein-, Fumar-, Itaconsäure oder -säureanhydriden abgeleiteten Polymeren mit Vinylestern, Vinylethern, Vinylhalogeniden, Phenylvinylderivaten, Acrylsäure und ihren Estern, wie Copolymere von Methylvinylether/monoverestertem Maleinsäureanhydrid, Copolymere von Methacrylsäure und Methylmethacrylat, Copolymere von Methacrylsäure und Ethylacrylat und Vinylacetat/Crotonsäure-Copolymere und mit Polyethylenglykol gepfropfte Vinylacetat/Crotonsäure-Copolymere.

7. Aerosolvorrichtung nach einem der vorhergehenden Ansprüche, **dadurch gekennzeichnet, dass** die aus einem anionischen Carboxylpolymer ohne Urethanmotiv ausgewählte(n) Verbindung(en) (A) in einer Menge von 0,5 bis 20 Gew.-%, noch besser von 1 bis 12 Gew.-%, bezogen auf das Gesamtgewicht der Zusammensetzung, vorliegen.

8. Aerosolvorrichtung nach einem der vorhergehenden Ansprüche, **dadurch gekennzeichnet, dass** der einwertige Alkohol vorzugsweise aus Ethanol, Isopropanol, n-Propanol, tert-Butanol oder deren Gemischen, vorzugsweise aus Ethanol, Isopropanol oder deren Gemischen ausgewählt ist, noch stärker bevorzugt Ethanol ist.

9. Aerosolvorrichtung nach einem der vorhergehenden Ansprüche, **dadurch gekennzeichnet, dass** die Zusammensetzung 1 bis 95 Gew.-% und vorzugsweise 10 bis 90 Gew.-% Wasser, bezogen auf das Gesamtgewicht der Zusammensetzung, umfasst.

10. Verwendung der Aerosolvorrichtung nach einem der vorhergehenden Ansprüche als Haarspray.

11. Frisierverfahren, **dadurch gekennzeichnet, dass** man die in der Aerosolvorrichtung nach einem der Ansprüche 1 bis 9 enthaltene Frisierzusammensetzung auf die nassen oder trockenen Haare sprüht.

## Claims

1. Two-compartment aerosol device, comprising:
a) in a first compartment, a styling composition comprising at least one compound (A) chosen from an anionic carboxylic polymer free of urethane units in an alcoholic or aqueous-alcoholic medium which comprises from 20% to 98% by weight, relative to the weight of said composition, of a C₁-C₄ monohydric alcohol, and
b) in a second compartment, a compressed gas chosen from air, nitrogen and carbon dioxide, and mixtures thereof,
provided that when the styling composition further comprises one or more additional fixing polymers, these are not polyurethanes.

2. Aerosol device according to Claim 1, **characterized in that** the compressed gas is air.

3. Aerosol device according to Claim 1 or 2, **characterized in that** the pressure of the compressed gas is between 1 and 12 bar.

4. Aerosol device according to Claim 3, **characterized in that** the pressure of the compressed gas is between 9 and 11 bar.

5. Device according to any one of the preceding claims, **characterized in that** the anionic polymer is chosen from:
A) copolymers of acrylic acid and of acrylamide, the sodium salts of polyhydroxycarboxylic acids;
B) copolymers of acrylic or methacrylic acid with a monoethylenic monomer such as ethylene, styrene, vinyl esters and acrylic or methacrylic acid esters, optionally grafted onto a polyalkylene glycol such as polyethylene glycol and optionally crosslinked;
C) crotonic acid copolymers, such as those comprising vinyl acetate or propionate units in their chain and optionally other monomers such as allyl esters or methallyl esters, vinyl ether or vinyl ester of a linear or branched saturated carboxylic acid with a long hydrocarbon-based chain, such as those containing at least 5 carbon atoms, it being possible for these polymers optionally to be grafted or crosslinked, or alternatively another vinyl, allyl or methallyl ester monomer of an α- or β-cyclic carboxylic acid;
D) copolymers of C4-C8 monounsaturated carboxylic acids or anhydrides chosen from:
- copolymers comprising (i) one or more maleic, fumaric or itaconic acids or anhydrides and (ii) at least one monomer chosen from vinyl esters, vinyl ethers, vinyl halides, phenylvinyl derivatives, acrylic acid and esters thereof, the anhydride functions of these copolymers optionally being monoesterified or monoamidated;
- copolymers comprising (i) one or more maleic, citraconic or itaconic anhydride units and (ii) one or more monomers chosen from allyl or methallyl esters optionally comprising one or more acrylamide, methacrylamide, α-olefin, acrylic or methacrylic ester, acrylic or methacrylic acid or vinylpyrrolidone groups in their chain,
the anhydride functions of these copolymers optionally being monoesterified or monoamidated;
E) polyacrylamides comprising carboxylate groups.

6. Aerosol device according to any one of Claims 1 to 5, **characterized in that** the anionic polymer is chosen from acrylic acid copolymers, such as acrylic acid/ethyl acrylate/N-tert-butylacrylamide terpolymers, copolymers derived from crotonic acid, such as vinyl acetate/vinyl tert-butylbenzoate/crotonic acid terpolymers and crotonic acid/vinyl acetate/vinyl neododecanoate terpolymers, polymers derived from maleic, fumaric or itaconic acids or anhydrides with vinyl esters, vinyl ethers, vinyl halides, phenylvinyl derivatives and acrylic acid and esters thereof, such as methyl vinyl ether/monoesterified maleic anhydride copolymers, copolymers of methacrylic acid and of methyl methacrylate, copolymers of methacrylic acid and of ethyl acrylate, vinyl acetate/crotonic acid copolymers, and vinyl acetate/crotonic acid copolymers grafted with polyethylene glycol.

7. Aerosol device according to any one of the preceding claims, **characterized in that** the compound(s) (A) chosen from an anionic carboxylic polymer free of urethane units are present in an amount ranging from 0.5% to 20% by weight and even better still from 1% to 12% by weight relative to the total weight of the composition.

8. Aerosol device according to any one of the preceding claims, **characterized in that** the monohydric alcohol is preferably chosen from ethanol, isopropanol, n-propanol and tert-butanol, or mixtures thereof, preferably from ethanol and isopropanol, or mixtures thereof, and even more preferentially is ethanol.

9. Aerosol device according to any one of the preceding claims, **characterized in that** the composition comprises from 1% to 95% and preferably from 10% to 90% by weight of water relative to the total weight of the composition.

10. Use of the aerosol device according to any one of the preceding claims, for a hair lacquer.

11. Styling process, **characterized in that** the styling composition contained in the aerosol device according to any one of Claims 1 to 9 is vaporized onto wet or dry hair.
